# EUROPEAN PATENT SPECIFICATION

(11) **EP 4 003 446 B1**
(45) Date of publication and mention of the grant of the patent: **07.06.2023**
(21) Application number: 20743696.5
(22) Date of filing: 23.07.2020
(51) Int. Cl.: A61M 1/00, A61M 1/06

(54) **MOTOR-DRIVEN MEDICAL SUCTION PUMP**
MOTORISCH ANGETRIEBENE MEDIZINISCHE SAUGPUMPE
POMPE ASPIRANTE MÉDICALE MOTORISÉE

(30) Priority: 25.07.2019 EP 19188341
(43) Date of publication of application: 01.06.2022
(73) Proprietor: Medela Holding AG, 6340 Baar (CH)
(72) Inventor: WALTER, Andreas, 8910 Affoltern am Albis (CH); MARBET, Regina, 4933 Rütschelen (CH)
(74) Representative: Grünecker Patent- und Rechtsanwälte PartG mbB
(86) International application number: PCT/EP2020/070865
(87) International publication number: WO 2021/013959

(56) References cited:
- WO-A1-2017/157691
- DE-A1-102009 048 880
- US-A1- 2019 083 688

## Description

The present invention relates to a motor-driven medical suction pump with a pump unit for generating a vacuum and with a housing which accommodates the pump unit therein. The pump unit has a vacuum opening for the negative pressure to be generated by the pump unit and at least one further opening communicating with the vacuum opening through the pump unit.

Such a motor-driven medical suction pump is known from CN 202 342 540 U1. In this prior art, the further opening is an air release opening from which air is discharged from the pump unit during cyclic operation of the pump unit to generate the negative pressure. The generation of negative pressure at the vacuum opening causes air to be expelled from the pump unit. For this purpose, the pump unit comprises the said air release opening.

The prior publications WO 2019/096700 A2 and WO 2006/032156 A1, which can be traced back to the applicant, reveal a pump unit with a vacuum opening, an outlet opening and a ventilation opening, wherein the ventilation opening can be connected via a ventilation valve to a vacuum-carrying vacuum line within the pump unit in order to reduce an excessive negative pressure. This reduction of the negative pressure within the pump unit is due to the fact that the motor-driven medical suction pump described in the prior art is a breast pump and that when breast milk is pumped from the female breast, a certain negative pressure level should not be undercut, as the sucking is otherwise perceived as painful and damage to the female breast may also have to be feared.

According to WO 2019/096700 A2, the pump unit is completely housed in a pump unit housing. The air release opening and the ventilation opening, respectively, are exposed within this pump unit housing, described in the prior art as a case. The pump unit housing is made up of several parts. Air exiting through the air release opening is to escape through slots provided in the area of the joint surface between two housing parts which form the pump unit housing. The pump unit housing, which completely encloses the pump unit, provides a certain degree of soundproofing.

According to CN 202 342 540 U1, the air release opening is connected via an air release hose to a sound damping chamber in which a sound absorbing foam is provided and which is equipped with several outlet openings.

Further medical suction pumps with sound damping means are known from DE 10 2009 048 880 A1, US 2019/0083688 A1 and WO 2017/157691 A1.

The present invention is based on the problem of indicating a motor-driven medical suction pump of the type mentioned above, which is easy to manufacture, compact in design and also provides effective sound damping.

To solve this problem, the present invention proposes a motor-driven medical suction pump with the features of claim 1. This pump differs from the generic motor-driven medical suction pump in that the sound damping chamber is tightly connected to and encloses the further opening.

The further opening in the sense of the present invention can be the air release opening. However, it can also be the ventilation opening according to WO 2019/096700 A2. In any case, the opening is an opening which communicates via the pump unit, i.e. through flow paths formed within the pump unit, with the vacuum opening and within the pump unit. The air release opening usually communicates with the vacuum opening via a pump chamber in which, for example, a piston is moved cyclically, wherein at least one adjustable valve can be provided between the vacuum opening and the air release opening. The ventilation opening according to WO 2019/096700 A2 communicates directly with a vacuum line, which leads to the vacuum opening, within the pump unit when the ventilation valve is open. Thus both the ventilation opening and the air release opening are further openings in the sense of the present invention.

The further opening in the sense of the present invention is in particular the air release opening of the pump unit, from which air is discharged from the pump unit during cyclic operation of the pump unit to generate the negative pressure. The sound that is here exiting from the pump unit is usually stronger than the sound emitted in the area of the ventilation opening.

The sound damping chamber according to the invention is tightly connected to and encloses the further opening. The sound damping chamber is thus connected directly to the further opening and not via a hose as is the case with the prior art according to CN 202 342 540 U1. The solution according to the invention thus permits a compact design.

By directly connecting the further opening to the sound damping chamber, the sound emitted from the pump unit in the area of the further opening enters the sound damping chamber directly and is effectively dampened there. According to the present invention, the sound damping chamber encloses the further opening, which means that the further opening and thus a certain area proportion of the outer casing of the pump unit is located within the sound damping chamber. This area proportion can be formed solely by an outlet nozzle, which can enclose and form the further opening, in particular the air release opening. The sound damping chamber can, however, also take up further area proportions of the pump unit housing. In any case, the further opening formed by the pump unit, preferably the air release opening is located in the sound damping chamber and is exposed due to the enclosing of the opening by this sound damping chamber directly in the sound damping chamber.

The further opening is tightly connected to the sound damping chamber. Here, tight means soundproof in the sense that the sound emitted through the further opening cannot escape unhindered from the further opening to the outside, but is first introduced into the sound damping chamber. The sound damping chamber sealingly accommodates, for example, a nozzle of the pump unit housing surrounding the further opening. Alternatively, the sound damping chamber can also be connected soundproof to a surface area of the pump unit housing in which the further opening is exposed.

The outlet opening of the sound damping chamber is usually exposed in the suction pump housing or on the outside of this housing. The outlet opening is usually not directly connected to another chamber or a hose.

A pump unit of the suction pump according to the invention is in particular a unit which comprises a motor and a work unit which is connected to the motor by means of a drive and which comprises at least one work chamber in which a negative pressure effective at the vacuum opening can be generated due to the drive power of the motor. Such a drive unit can, for example, be a piston connected to the motor shaft via an eccentric and sealed and held movable via a membrane sealed in the pump unit housing, as known for example from WO 2006/032156 A1.

The housing of the motor-driven medical suction pump can be the outer housing of the suction pump, on the outside of which operating elements for controlling the suction pump and/or a vacuum connection for connecting a suction hose leading to a breast cap to be put on the mother's breast and/or a plug for connecting a power current to the pump unit can be provided. It is true that the motor-driven medical suction pump according to the invention is in particular a suction pump as is normally used in a breast pump device for pumping human breast milk. However, the pump according to the invention is also suitable for other applications, for example as a drainage pump for sucking body fluids.

The outlet opening of the sound damping chamber is usually exposed in the housing of the suction pump according to the invention. The housing thus has an additional sound damping effect on any sound that may escape at the outlet opening. The housing is usually made up of several parts and usually has brackets to accommodate the pump unit or an energy store in the form of a battery or accumulator. The previously mentioned vacuum connection is usually exposed on the outside of the housing and is designed there in an adapted way to accommodate a plug of a suction hose, e.g. leading to the breast cap.

As already explained above, the sound damping chamber is connected directly to the pump unit, preferably to the housing of the pump unit. This results in a simple producibility of the suction pump according to the invention. This is because the sound damping chamber only has to be connected to the pump unit and can thus be prepared uniformly with the pump unit and handled during assembly.

With a view to good sound damping within the sound damping chamber, the further opening is provided opposite to the outlet opening. This creates the greatest possible distance between the further opening and the outlet opening, which has a favorable effect on the sound damping within the sound damping chamber. The further opening and the outlet opening can, for example, be located on a common axis parallel to the drive axis of a motor of the pump unit. An axisymmetric arrangement is particularly easy to implement in terms of production technology and regularly creates a relatively large distance of the outlet opening also from adjacent walls of the housing, which can provide secondary sound damping as described above.

Practical tests have shown that particularly good sound damping is achieved with a design in accordance with the present invention in which the further opening has a larger flow cross-section than the outlet opening. The diameter ratio between the diameter of the further opening, especially the air release opening, and the diameter of the outlet opening should be between 0.5 and 0.95, preferably 0.7.

In the case of the motor-driven medical suction pumps in question here, in particular for sucking breast milk from the female breast, it has proved to be advantageous to form the sound damping chamber with a volume of at least 6 ml. Below this limit value, the sound damping becomes increasingly inadequate. For reasons of compact design, it has proved to be advantageous to limit the volume of the sound damping chamber to 30 ml, preferably 29 ml. These specifications apply in particular to a motor-driven medical suction pump, which has a throughput of between 3 and 7 liters/min and/or can generate a negative pressure of approximately 250 to 300 mmHg on the suction side and at the vacuum opening.

According to a preferred further development of the present invention, a sound damping housing surrounding the sound damping chamber is made rigid. The sound damping housing is preferably made of plastic, especially ABS. But other plastics such as PA, PC, PP or PS are also conceivable for the design of the sound damping housing.

According to a preferred further development of the present invention, the sound damping chamber is formed by a hose piece. This hose piece is directly connected to the pump unit housing, usually to a nozzle which protrudes from the pump unit housing. With this design, the hose piece itself forms the sound damping chamber. Thus, one end of the hose is directly connected to the other opening, directly following it. The other end of the hose forms the outlet opening and is exposed in the housing. This outlet opening can be formed by the free, cut end of the hose piece. However, the outlet opening may also be formed by a plug inserted tightly into the free end of the hose piece to provide an outlet opening with a diameter different from the lumen of the hose.

A particularly easily producible and compact design of the present invention is given by a preferred design of the present invention, in which the sound damping chamber is fastened to the pump unit. The sound damping housing surrounding the sound damping chamber usually comprises fastening elements formed directly in one piece by a plastic housing, which interact with the pump unit, usually the pump unit housing. The sound damping chamber can be glued, screwed or welded to the pump unit. For easy installation, form-locking connecting elements connecting the damping chamber to the pump unit shall be preferred.

Thus, in accordance with a preferred further development of the present invention, it is proposed that a sound damping housing surrounding the sound damping chamber be locked in place with the pump unit. In this design, the sound damping housing is usually made of plastic together with locking hooks that fully or partially grip around the pump unit and lock in place therewith. The design of the plastic sound damping housing has the advantage that the locking hooks have the desired elasticity for locking and the corresponding resilience.

With regard to a simple tight abutment of the sound damping housing on the pump unit, it is preferable to provide the sound damping housing with a fastening flange, preferably to form such a fastening flange in one piece on the sound damping housing, which fastening flange tightly abuts on at least two surface sections, which are substantially at right angles to each other, of the pump unit, in particular the pump unit housing. With this design, at least two surface sections of a labyrinth seal between the sound damping housing and the pump unit are implemented, which ensure a good soundproof system. Preferably, several such surface sections extending at right angles to each other are arranged one behind the other and lie against a correspondingly designed contour of the fastening flange.

The pump unit according to the present invention is preferably made cylindrical, at least in sections. A cylindrical section is usually given by the motor of the pump unit, which is usually an electric motor. Preferably, the motor housing of this motor is followed by the housing of the work unit. This housing of the work unit can be realized with approximately the same diameter as the motor housing, thereby creating a pump unit which is cylindrical in sections and which can be accommodated in a space-saving way in the housing of the suction pump according to the invention. The housing of the work unit usually has a larger diameter than the motor housing, which results in a step against which a locking hook of the sound damping housing can be locked in order to connect this sound damping housing to the pump unit. The sound damping housing is also preferably made cylindrical, namely with approximately the same diameter as the housing of the work unit and/or the motor housing. This creates a sound-dampened pump unit with a cylindrical design, at least in sections, which can be accommodated in the housing in a space-saving manner.

According to another preferred design of the present invention, the sound damping housing surrounding the sound damping chamber is on the one hand made open and is closed by the pump unit. This design offers the possibility of a simple production of the sound damping housing by injection molding, because the sound damping housing is here designed as a pot which is open at one side and which can be easily demolded during injection molding. Locking hooks, which are integrally molded onto the injection molded part and interact with the pump unit to fasten the sound damping housing to the pump unit, protrude here preferably from the area of the sound damping housing surrounding the sound damping chamber at the circumference and at the end and forming the actual sound damping chamber. The tight connection of such a sound damping housing is preferably made via the previously discussed fastening flange, which forms a labyrinth seal with the outer contours of the pump unit.

On the other hand, an alternative design in which the sound damping housing completely surrounds the sound damping chamber and is only open to the outside via the further opening and the outlet opening offers the advantage of a sound damping chamber that is maximally closed and can therefore be optimally designed in terms of sound damping. The outlet opening is usually recessed as a simple bore in the wall of the sound damping chamber. The further opening is preferably located within a nozzle projecting from the pump unit, which is surrounded by the sound damping housing, so that the air release opening provided at the free end of the nozzle is located within the sound damping chamber and the sound damping housing tightly encloses the nozzle, preferably held by the nozzle. For this purpose, a bore of the sound damping chamber assigned to the nozzle is adapted to the outer circumferential surface of the nozzle in such a way that there is a tight pressing between the nozzle and the sound damping housing. The sound damping housing can be connected to the nozzle in a force-, form- or material-locking manner, in particular it can be glued, so that a permanent connection between the sound damping housing and the pump unit is ensured.

With a view to a compact arrangement of the essential components of the medical suction pump, it is proposed according to a preferred development of the present invention that the pump unit be mounted on a pump unit carrier. This pump unit carrier has a receptacle for an energy store. The energy store is preferably an elongated component which is provided parallel to the at least sectionally cylindrical pump unit with sound damping housing. The two parts can thus be mounted in parallel arrangement relative to each other on the pump unit carrier. The energy store and the pump unit together with the sound damping housing are matched to each other in such a way that they have approximately the same axial length. Thus, a vacuum hose laterally branches off from the pump unit at the height of the energy store, which vacuum hose is connected to the vacuum opening and connects said opening to a through-opening, which is usually exposed on the outside of the housing and is provided with the aforementioned vacuum connection, in order to connect there a plug of a suction hose, which leads, for example, to the breast cap, to the suction pump flowwise and mechanically.

In this design, a controllably adjustable ventilation opening is preferably connected to the vacuum hose in order to avoid an excessive negative-pressure level in the area of the vacuum line and thus in the area of the vacuum connection. Details of the function of such an adjustable or controllable ventilation opening are described in WO 2006/032156 A1.

A ventilation valve is usually provided for the adjustable ventilation opening, which ventilation valve is provided in the axial extension of the energy store and pump unit, respectively. This is because the parallel arrangement of the pump unit and the energy store is carried out with the aim of arranging these space-consuming components of the suction pump essentially over the entire width extension of the pump unit carrier. Thus, with a compact arrangement of the components in the axial extension of the energy store and pump unit, respectively, there is room for accommodating further components.

For the same reasons, it is proposed according to a preferred design of the present invention that the pump unit should be formed by an elongated body which comprises the sound damping chamber at its one end and a motor of the pump unit at its other end and that this unit should be mounted on the pump unit carrier in a substantially parallel arrangement with respect to the energy store, which pump unit carrier carries the aforementioned ventilation valve upstream of the energy store.

Further details and advantages of the present invention become apparent from the following description of an embodiment in conjunction with the drawing. In this drawing,
- Figure 1: shows a longitudinal sectional view of a pump unit with a first embodiment of a sound damping chamber;
- Figure 2: shows a longitudinal sectional view of a pump unit with a second embodiment of a sound damping chamber;
- Figure 3: shows a longitudinal sectional view of a pump unit with a third embodiment of a sound damping chamber;
- Figure 4: shows an exploded view of an embodiment of a motor-driven medical suction pump;
- Figure 5: shows a top view on the embodiment shown in Figure 4; and
- Figure 6: shows a perspective side view of the embodiment according to Figures 4 and 5.

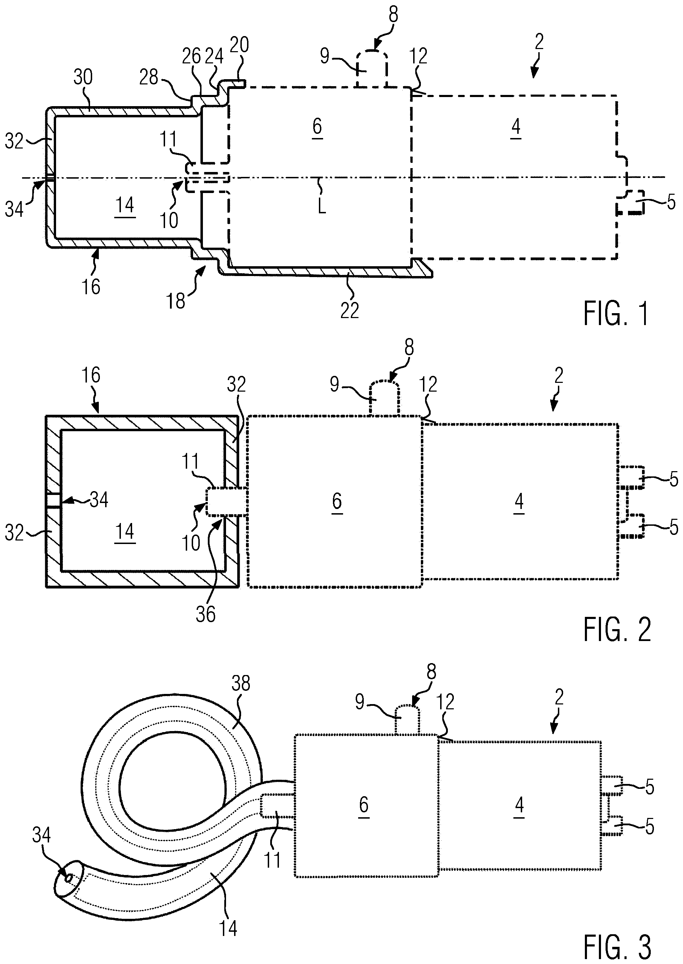

In Figures 1 to 3, reference sign 2 marks a pump unit which comprises an electric motor 4 and a work unit 6. The work unit 6 can comprise the components described in WO 2006/032156 A1 and may be constructed in a similar way. The electric motor 4 is made cylindrical, with electrical connection tongues 5 projecting from the free front side. Adjacent to the opposite front side is the work unit 6 which is also essentially of a cylindrical design and from which a vacuum nozzle 9, which surrounds a vacuum opening 8, projects in the circumferential direction and at which an air release opening 10 is provided opposite the electric motor 4 and is formed by an air release nozzle 11, which projects there from a front face of the work unit 6. The diameter of the electric motor 4 is slightly smaller than the diameter of the work unit 6. This creates a ring surface 12 between the two components 4, 6.

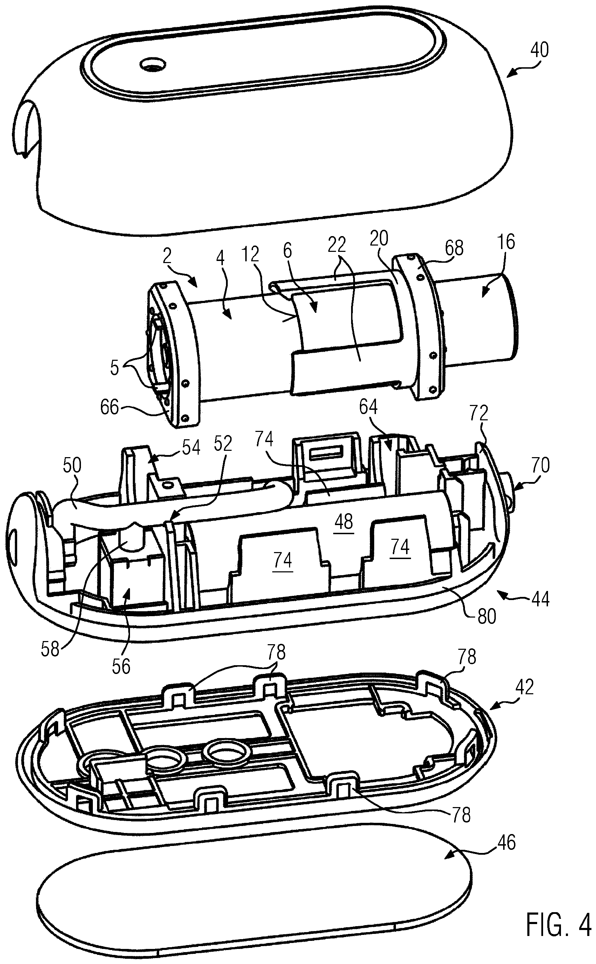

Figure 1 shows a first example of a sound damping chamber 14, which in this case is formed by a pot-shaped sound damping housing 16 open on one side, which is closed off by the work unit 6 opposite to the front face comprising the air release opening 10. The sound damping housing 16 comprises a fastening flange 18 with a multi-stepped course. The fastening flange 18 has an outer ring section 20 which surrounds the end of the outer circumferential surface of the work unit 6 and from which locking hooks 22 project, the hooks locking against the ring surface 12. As Figures 4 to 6 in particular show, several locking hooks 22, distributed in the circumferential direction, branch off from the outer ring section 12 and are connected in a form-locking manner to the pump unit 2 in the way mentioned above and as can be seen in Figure 1. The outer ring section 20 is connected via an outer radial section 24, an inner ring section 26 and an inner radial section 28 to a cylinder section 30, which surrounds the actual sound damping chamber 14 circumferentially and is provided with an end cap 32, which comprises an outlet opening 34. The fastening flange 18 with the stepped structure described above, the cylinder section 30 and the cap 32 are formed by a unitary plastic component which also forms the locking hooks 22. The plastic component is here injection-molded from ABS.

Figure 1 shows the housing of the work unit 6 in dash-dotted fashion. In this design of the work unit 6, only the outer ring section 20 and the outer radial section 24 rest against surface sections of the work unit 6 which extend at right angles to each other. However, the work unit 6 may also have a stepped course corresponding to the stepped course of the fastening flange 18, so that all surface sections of the fastening flange 18 formed from the inner radial section 28 to the outer ring section 20 lie against the work unit 6, thereby forming a very effective labyrinth seal via which the sound damping housing 16 lies tightly against the work unit 6. Thus the sound damping chamber 14 is substantially airtightly connected to the pump unit 2.

As Figure 1 shows, the outlet opening 34 has a smaller diameter than the air release opening 10. It is located together with the air release opening 10 on an axis L defined by the shaft of the electric motor 2.

Corresponding diameter ratios are also realized in the second example of a sound damping chamber according to Figure 2. Identical components are marked with identical reference signs. The sound damping chamber 14 is surrounded by a sound damping housing 16, which is made cylindrical and completely closed in the circumferential direction and has only on its opposite caps 32, which extend transversely to an axial direction of the cylindrical body, on the one hand the outlet opening 34 and on the other hand a receiving bore 36, which is adapted to the outer diameter of the air release nozzle 11, so that the sound damping housing 16 is sealed off from and held on this air release nozzle 11. The sound damping housing 16 is glued to the air release nozzle 11 or otherwise airtightly connected.

Figure 3 also shows a corresponding fastening of the sound damping chamber 14 to the pump unit 2. There, the sound damping chamber 14 is formed by the lumen of a hose 38 which is tightly pushed onto the air release nozzle 11 and can consist of a soft-elastic plastic, e.g. silicone. The free end of the hose 38 forms the outlet opening 34. This outlet opening 34, too, preferably has a smaller diameter than the air release opening 10 and also a smaller diameter than the diameter of the sound damping chamber 14. This reduced diameter can be achieved by means of a plug connected to the hose 38. The outlet opening is a free opening exposed in a housing of the pump.

Figures 4 to 6 show an embodiment of a motor-driven medical suction pump using a pump unit 2 with a sound damping housing 16 corresponding to the first embodiment according to Figure 1. Identical components are also marked here with identical reference signs.

The embodiment comprises a housing lid 40 and a housing bottom 42 which, when closed, accommodate a pump unit carrier 44 between them. Between the pump unit carrier 44 and the housing bottom 42, a printed circuit board assembly (not shown) with control elements may be provided; the control elements of this board are exposed against a cover marked with reference sign 46, as described in WO 2019/096700 A2. The elements 40 to 44 form a housing of the embodiment.

This laid-open publication provides further information on details of the embodiment, which have also been implemented here. Thus, reference can be made to the prior disclosure.

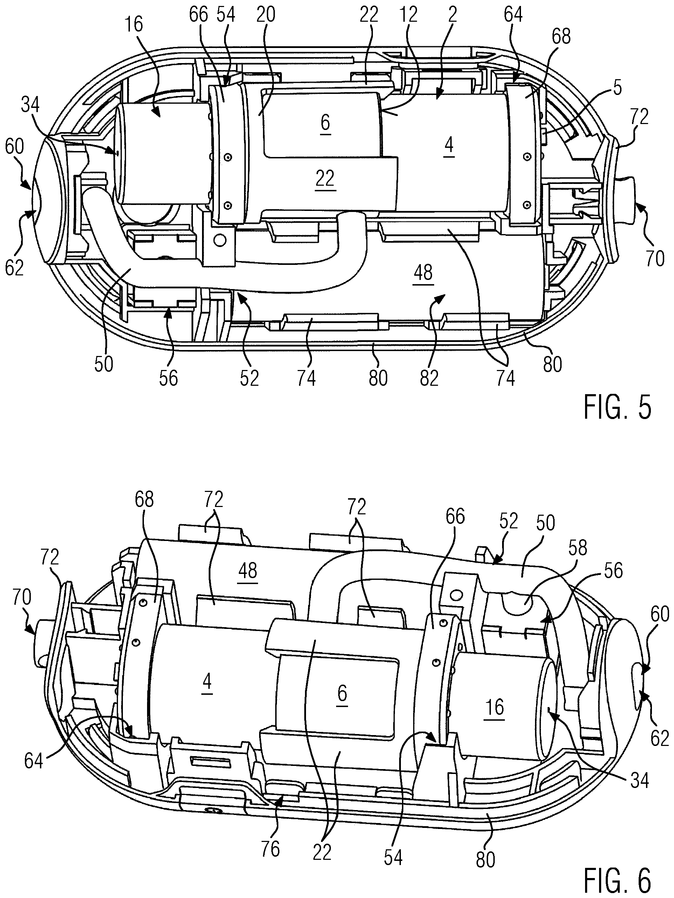

Deviating from the disclosure of WO 2019/096700 A2, an energy store 48 and the pump unit 2 are here provided on the pump unit carrier 44 in parallel arrangement. The pump unit 2 (without the sound damping chamber 14) has approximately the same axial length as the energy store 48 (see Figure 5). It can also be seen there that a vacuum hose marked with reference sign 50 and connected to the vacuum nozzle 9 first branches off radially from the pump unit 2 at approximately the middle height of the energy store 48 and is then led out in the axial direction of the energy store 48 and above it as well as parallel to it. The vacuum hose 50 is clamped in a hose receptacle 52, which is formed by a wall penetrating the pump unit carrier 44 in width direction, which forms a first pump unit receptacle 54 as an extension of the hose receptacle 52. Directly adjacent to the hose receptacle 52 is a ventilation valve 56, which is connected flowwise to the vacuum hose 50 via a ventilation line 58. The function of the ventilation valve is described in particular in WO 2006/032156 A1. The ventilation valve 58 prevents excessive negative pressure in the vacuum hose 50.

The free end of the vacuum hose 50 is connected to a vacuum connection 60 which is exposed in a through-opening 62 formed by the pump unit carrier 44. The corresponding design serves as a receptacle of a plug of a suction hose that leads to the chest cap. The vacuum connection 60, for example, can be made of a soft elastic plastic material such as TPE or silicone to seal the plug connection.

A second pump unit receptacle 64 protrudes from the bottom of the pump unit carrier 44. The two pump unit receptacles 54, 64 are located approximately at the same height as the axial ends of the energy store 48. They accommodate first and second bearing modules 66, 68, which were pushed onto the pump unit 2 prior to its assembly. For this purpose the first bearing module 66 has a stepped bore which forms a ring surface inside the first bearing module 66 for the front contact of the electric motor 4. A correspondingly stepped bore is also realized for the second bearing module 68. This stepped bore forms contact surfaces for the inner radial section 28 and the inner ring section 26. After insertion into the pump unit receptacles 54, 64, which are designed as sliding guides, the pump unit 2 together with the sound damping housing 16 is thus fixedly mounted on the pump unit carrier 44.

As Figures 5 and 6 show, the ventilation valve 56 is located at the level of the sound damping chamber 14. The sound damping chamber 14 has a slightly smaller diameter than the work unit 6, leaving sufficient space radially adjacent to the sound damping chamber 14 for the arrangement of the ventilation valve 56.

At the opposite front side of the pump unit carrier 44, a cable feed-through 70 protrudes from a front wall 72 which leaves between itself and the axial ends of the pump unit 2 and the energy store 48, respectively, sufficient space for accommodating cables that lead from the cable feed-through 7 to the printed circuit board (not shown) and from there to the connection tongues 8 of the electric motor 4.

All in all, the embodiment illustrates a compact arrangement of the suction pump components. The pump unit carrier 44 is designed as an injection-molded part and has, in addition to the receptacles 52, 54, 64 for the pump unit 2 and the vacuum hose 50, clamps 74 formed in one piece on the pump unit carrier 44 for fastening the energy store 48 and locking edges 76, which interact with the locking projections 78 there for fastening to the housing bottom 42, as well as an upstanding edge section 80 to which the housing lid 40 is connected. Between the clamps 74, a receptacle 82 is formed for the energy store 48.

### List of reference signs

- 2: pump unit
- 4: electric motor
- 5: connection tongue
- 6: work unit
- 8: vacuum opening
- 9: vacuum nozzle
- 10: air release opening
- 11: air release nozzle
- 12: ring surface
- 14: sound damping chamber
- 16: sound damping housing
- 18: fastening flange
- 20: outer ring section
- 22: locking hook
- 24: outer radial section
- 26: inner ring section
- 28: inner radial section
- 30: cylinder section
- 32: cap
- 34: outlet opening
- 36: receiving bore
- 38: hose
- 40: housing lid
- 42: housing bottom
- 44: pump unit carrier
- 46: cover
- 48: energy store
- 50: vacuum hose
- 52: hose receptacle
- 54: first pump unit receptacle
- 56: ventilation valve
- 58: ventilation line
- 60: vacuum connection
- 62: through-opening
- 64: second pump unit receptacle
- 66: first bearing module
- 68: second bearing module
- 70: cable feed-through
- 72: front wall
- 74: clamp
- 76: locking edges
- 78: locking projection
- 80: edge section
- 82: receptacle for the energy store

## Claims

1. Motor-driven medical suction pump with a pump unit (2) for generating a negative pressure with a housing (40, 42, 44) which accommodates the pump unit (2) therein, wherein the pump unit (2) comprises a vacuum opening (8) for the negative pressure to be generated by the pump unit (2) and at least one further opening (10) which communicates with the vacuum opening (8) through the pump unit (2) and which is connected to a sound damping chamber (14) comprising an outlet opening (34), wherein the sound damping chamber (14) is tightly connected to and encloses the further opening (10),
wherein the further opening (10) is provided opposite the outlet opening (34) and
**characterized in that** the further opening (10) has a larger flow cross-section than the outlet opening (34)

2. Motor-driven medical suction pump according to claim 1, **characterized in that** the further opening is an air release opening (10) of the pump unit (2).

3. Motor-driven medical suction pump according to one of the previous claims, **characterized in that** the sound damping chamber (14) has a volume of at least 6 ml, preferably not more than 30 ml.

4. Motor-driven medical suction pump according to one of the previous claims, **characterized in that** a sound damping housing (16) surrounding the sound damping chamber (14) is rigid, preferably made of a plastic, particularly preferably ABS.

5. Motor-driven medical suction pump according to one of the previous claims, **characterized in that** the sound damping chamber (14) is formed by a hose piece (38).

6. Motor-driven medical suction pump according to one of the previous claims, **characterized in that** the sound damping chamber (14) is fastened to the pump unit (2).

7. Motor-driven medical suction pump according to one of the previous claims, **characterized in that** a sound damping housing (16) surrounding the sound damping chamber (14) is locked to the pump unit (2).

8. Motor-driven medical suction pump according to one of the previous claims, **characterized in that** a sound damping housing (16) surrounding the sound damping chamber (14) comprises a fastening flange (18) which bears tightly against at least two surface sections of the pump unit (2) which extend substantially at right angles to each other.

9. Motor-driven medical suction pump according to one of the previous claims, **characterized in that** a sound damping housing (16) surrounding the sound damping chamber (14) is of a cylindrical design.

10. Motor-driven medical suction pump according to one of the previous claims, **characterized in that** a sound damping housing (16) surrounding the sound damping chamber (14) is made open on one side and is closed by the pump unit (2).

11. Motor-driven medical suction pump according to one of claims 1 to 9, **characterized in that** a sound damping housing (16) surrounding the sound damping chamber (14) completely surrounds the sound damping chamber (14) and is open outwards only via the further opening (10) and the outlet opening (34).

12. Motor-driven medical suction pump according to one of the previous claims, **characterized in that** the pump unit (2) is mounted on a pump unit carrier (44) which forms a receptacle (82) for an energy store (48), at the height of which a vacuum hose (50) branches off laterally from the pump unit (2), which vacuum hose connects the vacuum opening (8) to a through-opening (62) and to which a controllably adjustable ventilation opening is connected.

13. Motor-driven medical suction pump according to one of the previous claims, **characterized in that** the pump unit (2) is formed by an elongate body comprising the sound damping chamber (14) at its one end and a motor (4) of the pump unit (2) at its other end, and that an elongate energy store (48) extending substantially parallel to the pump unit (2) is mounted together with the pump unit (2) on a pump unit carrier (44) which carries a ventilation valve (56) upstream of the energy store (48) and adjusting a ventilation opening.

## Patentansprüche

1. Motorbetriebene medizinische Saugpumpe mit einem Pumpaggregat (2) zur Erzeugung eines Unterdrucks mit einem Gehäuse (40, 42, 44), das das Pumpaggregat (2) in sich aufnimmt, wobei das Pumpaggregat (2) eine Vakuumöffnung (8) für den von dem Pumpaggregat (2) zu erzeugenden Unterdruck und zumindest eine weitere, mit der Vakuumöffnung (8) durch das Pumpaggregat (2) kommunizierende Öffnung (10) aufweist, die mit einer eine Auslassöffnung (34) aufweisenden Schalldämpfungskammer (14) verbunden ist, wobei
die Schalldämpfungskammer (14) dicht an die weitere Öffnung (10) angeschlossen ist und diese umschließt,
wobei die weitere Öffnung (10) gegenüberliegend zu der Auslassöffnung (34) vorgesehen ist,
**dadurch gekennzeichnet,**
**dass** die weitere Öffnung (10) einen größeren Strömungsquerschnitt als die Auslassöffnung (34) hat.

2. Motorbetriebene medizinische Saugpumpe nach Anspruch 1, **dadurch gekennzeichnet, dass** die weitere Öffnung eine Entlüftungsöffnung (10) des Pumpaggregats (2) ist.

3. Motorbetriebene medizinische Saugpumpe nach einem der vorherigen Ansprüche, **dadurch gekennzeichnet, dass** die Schalldämpfungskammer (14) ein Volumen von mindestens 6 ml, bevorzugt nicht mehr als 30 ml hat.

4. Motorbetriebene medizinische Saugpumpe nach einem der vorherigen Ansprüche, **dadurch gekennzeichnet, dass** ein die Schalldämpfungskammer (14) umgebendes Schalldämpfungsgehäuse (16) steif, bevorzugt aus einem Kunststoff, besonders bevorzugt aus ABS ausgebildet ist.

5. Motorbetriebene medizinische Saugpumpe nach einem der vorherigen Ansprüche, **dadurch gekennzeichnet, dass** die Schalldämpfungskammer (14) durch ein Schlauchstück (38) gebildet ist.

6. Motorbetriebene medizinische Saugpumpe nach einem der vorherigen Ansprüche, **dadurch gekennzeichnet, dass** die Schalldämpfungskammer (14) an dem Pumpaggregat (2) befestigt ist.

7. Motorbetriebene medizinische Saugpumpe nach einem der vorherigen Ansprüche, **dadurch gekennzeichnet, dass** ein die Schalldämpfungskammer (14) umgebendes Schalldämpfungsgehäuse (16) mit dem Pumpaggregat (2) verrastet ist.

8. Motorbetriebene medizinische Saugpumpe nach einem der vorherigen Ansprüche, **dadurch gekennzeichnet, dass** ein die Schalldämpfungskammer (14) umgebendes Schalldämpfungsgehäuse (16) einen Befestigungsflansch (18) aufweist, der dicht an mindestens zwei, sich im Wesentlichen rechtwinklig zueinander erstreckenden Flächenabschnitten des Pumpaggregats (2) anliegt.

9. Motorbetriebene medizinische Saugpumpe nach einem der vorherigen Ansprüche, **dadurch gekennzeichnet, dass** ein die Schalldämpfungskammer (14) umgebendes Schalldämpfungsgehäuse (16) zylindrisch ausgebildet ist.

10. Motorbetriebene medizinische Saugpumpe nach einem der vorherigen Ansprüche, **dadurch gekennzeichnet, dass** ein die Schalldämpfungskammer (14) umgebendes Schalldämpfungsgehäuse (16) einseitig offen ausgebildet und durch das Pumpaggregat (2) verschlossen ist.

11. Motorbetriebene medizinische Saugpumpe nach einem der Ansprüche 1 bis 11, **dadurch gekennzeichnet, dass** ein die Schalldämpfungskammer (14) umgebendes Schalldämpfungsgehäuse (16) die Schalldämpfungskammer (14) vollumfänglich umgibt und lediglich über die weitere Öffnung (10) und die Auslassöffnung (34) nach außen offen ist.

12. Motorbetriebene medizinische Saugpumpe nach einem der vorherigen Ansprüche, **dadurch gekennzeichnet, dass** das Pumpaggregat (2) auf einem Pumpaggregatträger (44) montiert ist, der eine Aufnahme (82) für einen Energiespeicher (48) ausbildet, auf dessen Höhe seitlich von dem Pumpaggregat (2) ein Vakuumschlauch (50) abgeht, der die Vakuumöffnung (8) mit einer Durchgangsöffnung (62) verbindet und an den eine gesteuert stellbare Belüftungsöffnung angeschlossen ist.

13. Motorbetriebene medizinische Saugpumpe nach einem der vorherigen Ansprüche, **dadurch gekennzeichnet, dass** das Pumpaggregat (2) durch einen länglichen Körper gebildet ist, der an seinem einen Ende die Schalldämpfungskammer (14) und an seinem anderen Ende einen Motor (4) des Pumpaggregats (2) aufweist, und dass ein sich im Wesentlichen parallel zu dem Pumpaggregat (2) erstreckender länglicher Energiespeicher (48) zusammen mit dem Pumpaggregat (2) auf einem Pumpaggregatträger (44) montiert ist, der dem Energiespeicher (48) vorgelagert ein eine Belüftungsöffnung stellendes Belüftungsventil (56) trägt.

## Revendications

1. Pompe d'aspiration médicale entraînée par un moteur ayant une unité pompe (2) en vue de la génération d'une pression négative présentant un logement (40, 42, 44) qui accueille l'unité pompe (2) à l'intérieur, l'unité pompe (2) comprenant une ouverture de vide (8) pour la pression négative à générer par l'unité pompe (2) et au moins une ouverture supplémentaire (10) qui communique avec l'ouverture de vide (8) à travers l'unité pompe (2) et qui est raccordée à une chambre d'amortissement de son (14) comprenant une ouverture de sortie (34), la chambre d'amortissement de son (14) étant étroitement raccordée à, et enfermant, l'ouverture supplémentaire (10),
l'ouverture supplémentaire (10) étant disposée à l'opposé de l'ouverture de sortie (34) et **caractérisée en ce que** l'ouverture supplémentaire (10) présente une section transversale d'écoulement plus grande que l'ouverture de sortie (34).

2. Pompe d'aspiration médicale entraînée par un moteur selon la revendication 1, **caractérisée en ce que** l'ouverture supplémentaire est une ouverture (10) de libération d'air de l'unité pompe (2).

3. Pompe d'aspiration médicale entraînée par un moteur selon l'une des revendications précédentes, **caractérisée en ce que** la chambre d'amortissement de son (14) présente un volume d'au moins 6 ml, préférablement de pas plus de 30 ml.

4. Pompe d'aspiration médicale entraînée par un moteur selon l'une des revendications précédentes, **caractérisée en ce qu'**un logement d'amortissement de son (16) entourant la chambre d'amortissement de son (14) est rigide, préférablement constitué de plastique, de manière particulièrement préférable d'ABS.

5. Pompe d'aspiration médicale entraînée par un moteur selon l'une des revendications précédentes, **caractérisée en ce que** la chambre d'amortissement de son (14) est formée d'une pièce de tuyau (38).

6. Pompe d'aspiration médicale entraînée par un moteur selon l'une des revendications précédentes, **caractérisée en ce que** la chambre d'amortissement de son (14) est attachée à l'unité pompe (2).

7. Pompe d'aspiration médicale entraînée par un moteur selon l'une des revendications précédentes, **caractérisée en ce qu'**un logement d'amortissement de son (16) entourant la chambre d'amortissement de son (14) est verrouillé à l'unité pompe (2).

8. Pompe d'aspiration médicale entraînée par un moteur selon l'une des revendications précédentes, **caractérisée en ce qu'**un logement d'amortissement de son (16) entourant la chambre d'amortissement de son (14) comprend une collerette de fixation (18) qui se dirige fermement contre au moins deux sections de surface de l'unité pompe (2) qui s'étendent sensiblement sous des angles droits l'une par rapport à l'autre.

9. Pompe d'aspiration médicale entraînée par un moteur selon l'une des revendications précédentes, **caractérisée en ce qu'**un logement d'amortissement de son (16) entourant la chambre d'amortissement de son (14) est d'une conception cylindrique.

10. Pompe d'aspiration médicale entraînée par un moteur selon l'une des revendications précédentes, **caractérisée en ce qu'**un logement d'amortissement de son (16) entourant la chambre d'amortissement de son (14) est rendu ouvert sur un côté et est fermé par l'unité pompe (2).

11. Pompe d'aspiration médicale entraînée par un moteur selon l'une des revendications 1 à 9, **caractérisée en ce qu'**un logement d'amortissement de son (16) entourant la chambre d'amortissement de son (14) entoure entièrement la chambre d'amortissement de son (14) et est ouvert vers l'extérieur uniquement par l'intermédiaire de l'ouverture supplémentaire (10) et l'ouverture de sortie (34).

12. Pompe d'aspiration médicale entraînée par un moteur selon l'une des revendications précédentes, **caractérisée en ce que** l'unité pompe (2) est montée sur un support d'unité pompe (44) qui forme un réceptacle (82) pour un stockage d'énergie (48), à la hauteur duquel un tuyau de vide (50) se ramifie latéralement depuis l'unité pompe (2), lequel tuyau de vide raccorde l'ouverture de vide (8) à une ouverture traversante (62) et auquel est raccordée une ouverture de ventilation pouvant être ajustée de manière à pouvoir être contrôlée.

13. Pompe d'aspiration médicale entraînée par un moteur selon l'une des revendications précédentes, **caractérisée en ce que** l'unité pompe (2) est formée d'un corps allongé comprenant la chambre d'amortissement de son (14) au niveau de son extrémité et d'un moteur (4) de l'unité pompe (2) à son autre extrémité, et qu'un stockage d'énergie (48) allongé s'étendant sensiblement parallèle à l'unité pompe (2) est monté conjointement à l'unité pompe (2) sur un support d'unité pompe (44) qui porte une valve de ventilation (56) en amont du stockage d'énergie (48) et d'ajustement d'une ouverture de ventilation.
